# FASCICULE DE BREVET EUROPEEN

(11) **EP 0 737 101 B1**
(45) Date de publication et mention de la délivrance du brevet: **23.09.1998**
(21) Numéro de dépôt: 95903846.4
(22) Date de dépôt: 16.12.1994
(51) Int. Cl.: B01J 25/02, B01J 25/00

(54) **PROCEDE DE PREPARATION D'UN CATALYSEUR D'HYDROGENATION DE NITRILES EN AMINES ET APPLICATION DE CE CATALYSEUR EN HYDROGENATION**
VERFAHREN ZUR HERSTELLUNG EINES KATALYSATORS FÜR DIE HYDRIERUNG VON NITRILEN IN AMINEN UND VERWENDUNG DIESES KATALYSATORS IN DER HYDRIERUNG
METHOD FOR THE PREPARATION OF A CATALYST FOR THE HYDROGENATION OF NITRILES INTO AMINES AND USE OF SAID HYDROGENATING CATALYST

(30) Priorité: 28.12.1993 FR 9316007
(43) Date de publication de la demande: 16.10.1996
(73) Titulaire: RHODIA FIBER & RESIN INTERMEDIATES, 92408 Courbevoie Cédex (FR)
(72) Inventeur: BESSON, Michèle, F-01700 Les Echets (FR); CORDIER, Georges, F-69340 Francheville (FR); FOUILLOUX, Pierre, F-69300 Caluire-et-Cuire (FR); MASSON, Jacqueline, F-38320 EYBENS (FR)
(74) Mandataire: Vignally, Noel
(86) Numéro de dépôt international: FR9401477
(87) Numéro de publication internationale: WO9517960

(56) Documents cités:
- EP-A- 0 223 035
- GB-A- 2 104 794
- US-A- 4 429 159
- Scientific Papers I.P.C.R., Tokyo, vol. 55, pp. 105-108 (1961)
- Applied Catalysis, vol. 49, pp. 91-99 (1989)

## Description

La présente invention relève du domaine de l'hydrogénation catalytique de nitriles en amines et, plus particulièrement, de dinitriles tels que l'adiponitrile (ADN) en diamines telles que l'hexaméthylène diamine (HMD) ou en aminonitriles tels que l'aminocapronitrile.

Plus précisément, la présente invention concerne un procédé de préparation de catalyseur d'hydrogénation de nitriles en amines, de type Nickel de Raney dopé par au moins un élément métallique d'addition, sélectionné dans les colonnes IVB, VB et VIB de la classification périodique.

Il concerne également les catalyseurs obtenus par mise en oeuvre de ce procédé et elle a enfin pour objet une application de ces catalyseurs en hydrogénation catalytique de nitriles en amines et, plus précisément, mais non limitativement d'adiponitrile en hexaméthylènediamine.

Depuis des lustres, l'hydrogénation catalytique s'effectue à l'aide de Nickels de Raney, qui sont obtenus par lessivage alcalin d'alliages précurseurs Al-Ni riches en aluminium, le lessivage ayant normalement pour effet de provoquer l'élimination d'une grande partie de l'aluminium. Le catalyseur fini se présente sous forme d'agglomérats dé cristallites de Nickel finement divisées se caractérisant par une importante surface spécifique et un contenu résiduel en aluminium variable.

Classiquement, les paramètres qui permettent d'apprécier les performances d'un catalyseur, sont l'activité, la sélectivité et la stabilité dans le temps.

Pour tenter d'optimiser ces propriétés dans les catalyseurs Ni de Raney d'hydrogénation, il a été proposé de leur adjoindre des promoteurs ou dopants qui sont des éléments métalliques d'addition, choisis parmi les métaux de transition dans la classification périodique. (notamment fer, cobalt, chrome, molybdène, zirconium, tantale, titane).

Selon une première voie dite "métallurgique", le dopage ou l'incorporation de promoteur à des catalyseurs de type Nickel de Raney, s'opère au stade de l'alliage précurseur Ni-Al, avant attaque alcaline. L'introduction du dopant dans l'alliage s'effectue à l'état fondu.

L'article de BN TYUTYUNNIKOV, "The Soviet Chemical Industries, N° 6, Juin 1971, pages 380 à 382", de même que l'écrit de FREIDLING et al, "Russian Chemical Rewiews, Vol. 33, N° 6, Juin 1964, pages 319 à 329", sont des références bibliographiques antérieures qui se rapportent à des catalyseurs Ni de Raney dopés avec au moins un métal de transition, par voie métallurgique. D'une manière générale, ces catalyseurs connus ne sont pas des plus satisfaisants en ce qui concerne l'activité, la sélectivité, et la stabilité dans le temps. Ainsi, les catalyseurs divulgués dans TYUTYUNNIKOV et al semblent relativement spécifiques des mononitriles dérivés des acides gras de 7 à 20 atomes de carbone. Mais cela n'est pas un gage d'efficacité en ce qui concerne, par exemple, les dinitriles.

Les catalyseurs Ni de Raney dopés et décrits par FREIDLING et al, sont actifs sur les dinitriles, mais leur sélectivité vis-à-vis de ces substrats reste faible. Enfin, les conditions réactionnelles d'hydrogénation requises pour ces catalyseurs (Ni/Al/Ti) ne sont pas idéales sur le plan industriel : température de 140 à 180°C, pression en hydrogène de 140 bars et milieu réactionnel constitué de n-butanol + ammoniac.

On connaît, par ailleurs, une seconde voie de dopage de catalyseur d'hydrogénation de type Nickel de Raney, décrite notamment par TAKAGI et YAMANAKA dans "Scientific Papers I.P.C.R.- TOKYO, 55, (1961), 105-108".

Ces auteurs se proposent d'améliorer l'activité et la sélectivité de catalyseurs de type Nickel de Raney par addition de divers sels métalliques (nitrates de cuivre de cobalt ou de chrome, molybdate d'ammonium, tungstate de sodium, chlorure ferrique) au cours de l'étape d'attaque alcaline de l'alliage précurseur. Le nitrile à hydrogéner testé dans ces expériences est l'acétonitrile. D'après ce document, les catalyseurs dopés mis en oeuvre auraient une activité accrue par rapport à leurs homologues non dopés. Ces constats sont sujets à caution car ils sont effectués sur la seule base de l'observation visuelle de courbes d'évolution de la consommation en hydrogène.

En outre, il faut savoir que l'incorporation de ces sels métalliques dans le milieu de lessivage alcalin est susceptible de gêner le processus d'obtention du catalyseur.

T. KOSCIELSKI et al ont divulgué, quant à eux, dans "Applied Catalysis, 49 (1989) 91-99 ESP BV", le dopage de Ni de Raney à l'aide de solutions de Cl₃Cr en milieu alcalin (NaOH), de manière à déposer in fine du Cr(OH)₃ sur le catalyseur. Ce dernier est actif dans l'hydrogénation du cyclohexène, de l'acétophénone et de glucose. Cet enseignement ne s'applique donc pas à l'hydrogénation des nitriles en amines. Les catalyseurs dopés Cr décrits dans ce document antérieur ne sont, en effet, pas performants dans ce type d'hydrogénation.

Dans cet état de la technique, l'un des objectifs essentiels de la présente invention est de fournir un procédé de préparation de catalyseurs de type Ni de Raney dopés, pour l'hydrogénation catalytique de nitriles en amines et de préférence de dinitriles en diamines ou en aminonitriles.

Un autre objectif de l'invention est de fournir un procédé de préparation d'un tel catalyseur qui soit commode à mettre en oeuvre et économique.

Un autre objectif de l'invention est de fournir un procédé de préparation de catalyseurs Ni de Raney dopés, qui soient actifs, sélectifs et stables, dans le cadre de l'hydrogénation de nitriles en amines et de préférence de dinitriles en diamines ou en aminonitriles.

Un autre objectif de l'invention est de fournir un procédé d'hydrogénation faisant application de ces catalyseurs et permettant notamment d'atteindre des sélectivités en hexaméthylènediamine supérieures à 95 % à partir d'adiponitrile.

Pour atteindre ces objectifs, la Demanderesse a eu le mérite de mettre en évidence, de façon tout à fait surprenante et inattendue, que le dopage en éléments métalliques de transition du Ni de Raney doit avantageusement être effectué sur le catalyseur fini à l'aide d'une solution dudit élément métallique.

La présente invention concerne ainsi un procédé de préparation de catalyseurs d'hydrogénation de nitriles en amines de type Nickel de Raney dopé par au moins un élément métallique d'addition, sélectionné parmi le chrome et le titane, caractérisé en ce qu'il consiste à mettre en suspension du Nickel de Raney dans une solution acide de l'élément métallique d'addition.

Une telle disposition de procédé favorise l'incorporation de l'élément métallique d'admission sur le catalyseur. Sans vouloir être lié par la théorie, il semble, en effet, avantageux que le nickel soit légèrement attaqué par des ions H⁺ de la solution acide.

Au sens de l'invention, le terme acide correspond à une solution dont le pH est inférieur ou égal à 7 et, de préférence, supérieur ou égal à 0,5.

Un pH compris entre 1 et 4 semble être particulièrement approprié.

En d'autres termes, l'acidité souhaitable est celle correspondant à une normalité comprise entre 0,3 et 0,5 N.

Cette acidité peut être apportée par l'élément dopant en tant que tel, par un vecteur de cet élément dopant ou bien encore par un adjuvant acidogène et/ou acide complémentaire.

Conformément à l'invention, il est préférable que l'élément dopant ou l'élément métallique d'addition mis en oeuvre, se présente sous forme d'oxydes ou de sels, de préférence d'acide minéral ou d'acide organique.

Par sel d'acide minéral, on désigne tous les ions conjugués d'acides forts (tels que par exemple HCl, H₂SO₄, NO₃H) ou d'acides minéraux faibles tels que les phosphates acides.

A titre d'exemples de sels d'acides organiques, on peut citer : les acétates, les phtalates, les adipates, les citrates, les tartrates, les malonates.

Les oxydes constituent également des moyens supports du dopant, en vue de son incorporation au catalyseur Nickel de Raney. Il peut s'agir de monoxydes, dioxydes, trioxydes ou autres.

Les chlorures, dont notamment TiCl₃, et les oxydes sont particulièrement préférés.

En complément ou à la place du vecteur du dopant ou du dopant lui-même, pour la fourniture d'acidité au milieu, il est possible, conformément à l'invention, de mettre en oeuvre un additif acidogène et/ou acide comme par exemple HCl, H₂SO₄, H₃PO₄, CH₃COOH...etc. Il s'agit là d'une démanche simple d'ajustement d'acidité, parfaitement à la portée de l'homme du métier.

Il est à considérer que la nature acide du milieu de préparation du catalyseur Ni de Raney dopé correspond à une forme préférée, mais non limitative, de mise en oeuvre du procédé selon l'invention.

L'adsorption d'éléments dopants sur le catalyseur NiAl, selon les modalités ci-dessus permet de conduire à une unité catalytique Ni/Al/dopant particulièrement active, sélective vis-à-vis des nitriles et, en particulier, vis-à-vis des dinitriles tels que l'adiponitrile.

Cette unité catalytique est également très stable dans le temps et n'engendre que de très faibles quantités d'impuretés d'hydrogénation. Ceci est particulièrement intéressant car, dans certains cas, ces impuretés sont très difficiles à séparer de l'amine visée. A titre d'exemple de telles impuretés, on peut évoquer le diaminocyclohexane, composé qui possède sensiblement la même température d'ébullition que l'hexaméthylènediamine résultant de l'hydrogénation de l'adiponitrile.

Selon une autre disposition avantageuse du procédé selon l'invention, la mise en suspension est suivie d'un lavage, de préférence à l'eau, du nickel de Raney au moins en partie dopé par l'élément métallique d'addition, l'une au moins de ces deux opérations étant éventuellement renouvelée n fois, n étant avantageusement compris entre 1 et 5.

Pour parfaire le dopage du catalyseur Ni de Raney mis en suspension dans la solution, de préférence acide, de dopant, il est donc important de bien le laver à l'eau, en répétant le lavage jusqu'à obtenir la limpidité et la neutralité des eaux de lavage.

Plusieurs mises en suspension successives du Ni de Raney dans la solution de dopant peuvent également être envisagées, dans le cadre du procédé selon l'invention.

Le catalyseur de Raney dopé obtenu est recueilli après lavage puis est stocké, de préférence, dans une base forte, comme par exemple NaOH, KOH ou d'autres hydroxydes alcalins.

Conformément à une variante de l'invention, on soumet le Nickel de Raney dopé ou non et en suspension dans une solution d'au moins un élément métallique d'addition, à un traitement de lessivage alcalin, effectué à une température supérieure ou égale à la température ambiante, de préférence comprise entre 50°C et 200°C et, plus préférentiellement encore, comprise entre 80°C et 120°C, ce lessivage étant éventuellement effectué sous une pression en hydrogène comprise entre 0,1 et 20 MPa, de préférence entre 1 et 5 MPa et, plus préférentiellement encore, entre 1,5 et 3 MPa.

En pratique, ce traitement complémentaire consiste à placer le catalyseur dopé ou non dans une solution de d'hydroxyde alcalin (tel que soude ou analogue), à chauffer jusqu'à environ 100°C sous agitation et sous une pression en hydrogène d'environ 2,5 MPa.

Après cette attaque alcaline à haute température, le catalyseur est rincé avec des solutions d'hydroxyde alcalin de titres décroissants, puis avec de l'eau jusqu'à atteindre la neutralité des eaux de lavage.

De la même façon que précédemment, le stockage du catalyseur Ni/Al/dopant s'effectue dans une base forte telle que la soude ou analogue (1N).

De façon privilégiée, le titane est adopté à titre d'élément métallique d'addition. L'un des vecteurs appropriés correspondant est le TiCl₃.

Il est apparu particulièrement avantageux d'effectuer des dopages de catalyseur Ni de Raney, jusqu'à obtenir des rapports dopant/Ni compris entre 0,5 % et 5 %, de préférence entre 1 % et 4 % et, plus préférentiellement encore, entre 1, 2 % et 3 % en poids.

Selon un autre mode de mise en oeuvre du procédé de préparation du catalyseur conforme à l'invention, il est envisageable d'incorporer deux éléments dopants de nature différente, par exemple, le titane et le chrome.

La présente invention a également pour objet un procédé d'hydrogénation catalytique de nitriles en amines, dans lequel on fait application du catalyseur Ni de Raney dopé obtenu par le procédé de l'invention décrit ci-dessus.

Grâce au catalyseur issu du procédé selon l'invention, l'hydrogénation de nitriles en amines se déroule particulièrement bien et permet d'atteindre des performances d'activité et de sélectivité tout à fait satisfaisantes. On note, de surcroît, que la production d'impuretés reste limitée.

La préparation d'un catalyseur Ni de Raney dopé et son application à l'hydrogénation catalytique de nitriles en amines, concernent plus particulièrement des dinitriles de formule (I) :

NC―R―CN (I)

dans laquelle R représente un groupement alkylène ou alcénylène, linéaire ou ramifié, ayant de 1 à 12 atomes de carbone, ou un groupement arylène ou aralkylène ou aralcénylène substitué ou non.

De préférence, on met en oeuvre dans le procédé de l'invention des dinitriles de formule (I) dans laquelle R représente un radical alkylène, linéaire ou ramifié ayant de de 2 à 6 atomes de carbone.

A titre d'exemples de tels dinitriles, on peut citer notamment l'adiponitrile, le méthylglutaronitrile, l'éthylsuccinonitrile, le malononitrile, le succinonitrile et le glutaronitrile et leurs mélanges, notamment les mélanges d'adiponitrile et/ou de méthylglutaronitrile et'ou d'éthylsuccinonitrile qui proviennent d'un même procédé de synthèse de l'adiponitrile.

En pratique, le cas où R = (CH₂)₄ représente un débouché prépondérant car cela correspond à la transformation de l'ADN :
- soit en HMD, diamine qui constitue un des monomères de base de la fabrication de polyamide-6,6
- soit en aminocapronitrile qui permet d'obtenir du caprolactame précurseur du polyamide-6.

L'introduction du substrat nitrile, par exemple l'adiponitrile, dans le milieu réactionnel se fait en respectant une concentration comprise entre 0,001 % et 30 % en poids par rapport au poids total (p/p) du milieu réactionnel et de préférence entre 0,1 % et 20 % p/p.

De façon privilégiée, la base forte mise en oeuvre est choisie parmi les composés suivants : LiOH, NaOH, KOH, RbOH, CsOH et leur mélanges.

En pratique, on utilise préférentiellement NaOH et KOH, pour un bon compromis performance-prix, bien que RbOH et CsOH donnent des résultats encore meilleurs.

Le milieu réactionnel d'hydrogénation est de préférence liquide. Il contient au moins un solvant apte à solubiliser le substrat nitrile à hydrogéner, sachant que cette transformation s'opère mieux lorsque ledit substrat se trouve en solution.

Suivant une modalité intéressante du procédé selon l'invention, on utilise un milieu réactionnel liquide au moins partiellement aqueux. L'eau est généralement présente dans une quantité inférieure ou égale à 50 %, avantageusement inférieure ou égale à 20 % en poids par rapport au milieu réactionnel total. Plus préférentiellement encore, la teneur en eau du milieu réactionnel est comprise entre 0,1 et 15 % en poids par rapport à l'ensemble des constituants dudit milieu.

En complément ou en substitution à l'eau, on peut prévoir au moins un autre solvant, du type alcool et/ou amide. Les alcools qui conviennent plus particulièrement sont par exemple le méthanol, l'éthanol, le propanol, l'isopropanol, le butanol, les glycols, tels que l'éthylène et/ou le propylène glycol, des polyols et/ou des mélanges desdits composés.

Dans le cas où le solvant est constitué par un amide, il peut s'agir par exemple du diméthylformamide ou du diméthylacétamide.

Lorsqu'il est employé avec l'eau, le solvant, de préférence alcoolique, représente de deux à quatre parties en poids pour une partie en poids d'eau et de préférence trois parties pour une partie d'eau.

Selon une autre caractéristique préférée de l'invention, on incorpore de l'amine, dont la préparation est visée par le procédé, au sein du milieu réactionnel. Il s'agit par exemple d'hexaméthylènediamine, lorsque le substrat nitrile est l'adiponitrile.

La concentration de l'amine visée dans le milieu réactionnel est avantageusement comprise entre 50 % et 99 % en poids par rapport à la totalité du solvant inclus dans ledit milieu réactionnel et, plus préférentiellement encore, est comprise entre 60 % et 99 % en poids.

La quantité de base dans le milieu réactionnel varie en fonction de la nature du milieu réactionnel.

Dès lors que le milieu réactionnel ne contient que de l'eau et de l'amine visée, à titre de milieu solvant liquide, la quantité de base est avantageusement supérieure ou égale à 0,1 mol/kg de catalyseur, de préférence comprise entre 0,1 et 2 mol/kg de catalyseur et plus préférentiellement encore entre 0,5 et 1,5 mol/kg de catalyseur.

Dans le cas où le milieu réactionnel comprend de l'eau et un alcool et/ou un amide, la quantité de base est supérieure ou égale à 0,05 mol/kg de catalyseur, est comprise de préférence entre 0,1 et 10,0 mol/kg et plus préférentiellement encore entre 1,0 et 8,0 mol/kg.

Une fois arrêtée la composition du milieu réactionnel et le choix du catalyseur, on procède à un mélange de ces deux éléments, puis on chauffe ce mélange à une température réactionnelle inférieure ou égale à 150°C, de préférence inférieure ou égale à 120°C et, plus préférentiellement encore, inférieure ou égale à 100°C.

Concrètement, cette température est comprise entre la température ambiante (20°C environ) et 100°C.

Préalablement, simultanément ou postérieurement au chauffage, l'enceinte réactionnelle est amenée à la pression en hydrogène convenable, c'est-à-dire, en pratique, comprise entre 0,10 et 10 MPa.

La durée de la réaction est variable en fonction des conditions réactionnelles et du catalyseur.

Dans un mode de fonctionnement discontinu, elle peut varier de quelques minutes à plusieurs heures.

Dans un mode de fonctionnement continu, qui est parfaitement envisageable pour le procédé selon l'invention, la durée n'est évidemment pas un paramètre figeable.

Il est à noter que l'homme du métier peut moduler la chronologie des étapes du procédé selon l'invention, selon les conditions opératoires. L'ordre donné ci-avant ne correspond qu'à une forme préférée, mais non limitative, du procédé selon l'invention.

Les autres conditions qui régissent l'hydrogénation (en mode continu ou discontinu) conforme à l'invention, relèvent de dispositions techniques traditionnelles et connues en elles-mêmes.

Les exemples qui suivent de préparation de catalyseurs Ni de Raney dopés et d'hydrogénation d'ADN en HMD, permettront de mieux comprendre l'invention. Ils feront également ressortir tous les avantages de l'invention et certaines de ses variantes.

### EXEMPLES

### Exemple 1 : Préparation d'un catalyseur Ni/Al/Ti, désigné ci-après par la référence A

Dans une fiole jaugée de 25 ml remplie d'argon, on prépare une solution violette contenant 1,8 % de TiCl₃ dans HCl 1,2 %.

On prélève environ 5 g de Ni de Raney non dopé obtenu de manière classique par lessivage sodique d'un alliage précurseur commercial Ni/Al 50/50 en poids. Il est rincé avec des portions de 20 ml d'eau jusqu'à neutralité des eaux de lavage. On pèse dans un pycnomètre 3,44 g de ce catalyseur humide qui est placé dans un ballon de 100 ml. Le ballon est purgé à l'argon. On introduit alors 20 ml de la solution de TiCl₃ sur le catalyseur. Le milieu réactionnel est placé sous courant d'argon et agité pendant 1 heure. La solution devient verte très rapidement.

Au bout d'une heure le catalyseur A est lavé à l'eau jusqu'à limpidité et neutralité des eaux de lavage. Ce catalyseur A ainsi dopé au titane est stocké dans la soude 1N.

Le catalyseur A a un rapport pondéral Ti/Ni de 1,6 %.

### Exemple 2 : Hydrogénation d'adiponitrile (ADN) en hexaméthylènediamine (HMD) avec le catalyseur A

On prélève et on lave 4 fois avec environ 20 ml d'eau (jusqu'à neutralité des eaux de lavage) le catalyseur A de l'exemple 1. Dans un pycnomètre, on pèse exactement environ 0,4 g de ce catalyseur.

A l'aide d'une seringue on introduit dans une ampoule d'injection, reliée à un autoclave, par une canalisation étanche, 6 g d'adiponitrile. L'ampoule est fermée, purgée et on établit une pression de 2,5 MPa d'hydrogène.

Dans l'autoclave doté d'un système d'agitation on place 525 µl de soude 2N, 1475 µl d'eau et, après l'avoir rempli d'argon, 40 g de solvant constitué d'HMD, d'éthanol (EtOH) et d'eau (63/31,5/5,5 en poids respectivement). On se trouve ainsi dans un milieu à 0,1 % en poids de NaOH par rapport à la masse totale de solvant.

On introduit alors les 0,4 g de catalyseur dans l'autoclave, qui est ensuite soigneusement fermé et purgé 2 fois à l'azote. On introduit alors 2,5 MPa d'hydrogène et chauffe à 80°C. Après avoir atteint cette température, l'agitation est lancée et l'adiponitrile contenu dans l'ampoule d'injection est coulé dans l'autoclave. On détecte la fin de réaction par la stabilisation de la consommation d'hydrogène.

La réaction dure 47 minutes. L'analyse chromatographique de l'hydrogénat permet de calculer le rendement de la réaction.

La sélectivité (S) en HMD en pourcentage est donnée par la relation : 100 - somme des sélectivités des sous-produits. En effet l'HMD étant mise en oeuvre dans le solvant réactionnel, elle ne peut pas être dosée directement de manière très précise. Par contre il a été vérifié que les sous-produits sont globalement tous identifiés.

Les sélectivités en chacun des sous-produits sont représentées par le pourcentage molaire du sous-produit formé par rapport à l'ADN transformé. Dans tous les exemples et essais comparatifs effectués, le taux de transformation de l'ADN (ainsi que celui de l'aminocapronitrile intermédiaire) est de 100 %.

Les sélectivités sont les suivantes : HMD = 97,18 %; HMI (Hexaméthylèneimine) = 0,38 % ; DCH (Diaminocyclohexane) = 0,04 % ; AMCPA (Aminométhylcyclopentylamine) = 0,05 % ; NEtHMD (N-éthylhexaméthylènediamine) = 0,07 % : BHT (Bis-hexaméthylènetriamine) = 2,27 %.

### Exemple 3 : Préparation d'un catalyseur Ni/Al/Ti, désigné ci-après par la référence A₁

Une partie du catalyseur A de l'exemple 1 est prélevée, placée avec 30 ml de soude 6N dans un réacteur de 150 ml. Le milieu est chauffé à 100°C sous 2,5 MPa d'hydrogène sous agitation pendant 1 heure.

Le catalyseur A₁ obtenu est alors rinçé avec 10 ml de soude 3N, 2N, 1N et 3 fois avec 10 ml d'eau, jusqu'à la neutralité des eaux de lavage. Il est ensuite stocké dans la soude 1N.

### Exemple 4 : Hydrogénation d'ADN en HMD avec A₁

Le catalyseur A₁ de l'exemple 3 est mis en oeuvre comme dans l'exemple 2. La réaction dure 23 minutes. L'analyse chromatographique de l'hydrogénat permet de calculer les sélectivités suivantes : HMD = 96,81 % ; HMI = 0,28 % ; DCH = 0,33 % ; AMCPA = 0,05 % ; NEtHMD = 0,05 % : BHT = 2,04 %.

### Exemple 5 : Préparation d'un catalyseur Ni/Al/Cr, désigné ci-après par la référence B.

Dans une fiole jaugée de 25 ml on pèse 2,22 g de CrCl₃ et on complète à 25 ml avec de l'eau.

Dans un ballon, on place 5 g de Ni de Raney non dopé de l'exemple 1 avec 10 ml d'eau et 10 ml de la solution de CrCl₃. Le milieu est agité pendant 75 min. Le catalyseur B est alors lavé avec de l'eau puis à nouveau placé dans le ballon dans 10 ml d'eau et 10 ml de la solution de CrCl₃ pendant 75 min. Finalement on récupère le catalyseur B et on le lave jusqu'à neutralité avec 6 fois 50 ml d'eau et on le stocke dans de la soude 1N.

Le catalyseur B a un rapport pondéral Cr/Ni de 3,5 %.

Une partie de ce catalyseur B est prélevée, placée dans 30 ml de soude 6N dans un réacteur de 150 ml, puis elle est chauffée à 100°C sous agitation et sous 2,5 MPa d'hydrogène pendant 1 heure. On récupère le catalyseur B₁ ainsi traité et on le rince avec 10 ml de soude 3N, puis de soude 2N, puis de soude N et enfin avec 3 fois 10 ml d'eau jusqu'à neutralité des eaux de lavage. Il est ensuite stocké dans de la soude N.

### Exemple 6 : Hydrogénation d'ADN en HMD avec B₁

Le catalyseur B₁ de l'exemple 5 est mis en oeuvre comme dans l'exemple 2. La réaction dure 56 minutes. L'analyse chromatographique de l'hydrogénat permet de calculer les sélectivités suivantes : HMD = 95,24 %; HMI = 0,66 %; DCH = 0,05 % ; AMCPA = 0,03 % ; NEtHMD = 0,08 % ; BHT = 2,24 %.

### Exemple 7 : Préparation d'un catalyseur Ni/Al/Cr désigné ci-après par la référence C

Dans une fiole jaugée de 25 ml on pèse 0,85 g de CrO₃ et on complète à 25 ml avec de l'eau. On obtient une solution orange de CrO₃ de concentration 0.34 mol.l⁻¹.

Dans un ballon on place 5 g de Ni de Raney non dopé de l'exemple 1 dans 10 ml d'eau, 10 ml de la solution de CrO₃. Le milieu est agité pendant 75 min. Le catalyseur est alors lavé avec de l'eau, puis mis à nouveau dans le ballon dans 10 ml d'eau et 10 ml de la solution de CrO₃ pendant 75 min. Le catalyseur C ainsi traité 2 fois avec la solution de trioxyde de chrome, est maintenant transféré avec 30 ml de soude 6N dans un réacteur, chauffé à 100°C sous agitation et sous 2,5 MPa d'hydrogène pendant 1 heure. Il est récupéré, rincé avec 10 ml de soude 3N, puis de soude 2N, puis de soude N et enfin avec 3 fois 10 ml d'eau. Il est ensuite stocké dans de la soude N.

Le catalyseur C a un rapport pondéral Cr/Ni de 2,1 %.

### Exemple 8 : Hydrogénation d'ADN en HMD avec C

Le catalyseur C de l'exemple 7 est mis en oeuvre comme dans l'exemple 2. La réaction dure 1 heure 50 minutes. L'analyse chromatographique de l'hydrogénat permet de calculer les sélectivités suivantes : HMD = 96,13 % ; HMI = 1,05 % ; DCH = 0,09 % ; AMCPA = 0,03 % ; NEtHMD = 0,05 % ; BHT = 1,79 %.

### Exemple 9 : Préparation d'un catalyseur Ni/Al/Cr désigné ci-après par la référence D

2,15 g de Ni de Raney non dopé de l'exemple 1 sont placés dans un autoclave avec 3 ml d'eau, 4,5 ml de la solution de CrO₃ préparée comme dans l'exemple 7 et 30 ml de soude 6N. L'ensemble est chauffé à 100°C sous 2,5 MPa d'hydrogène et sous agitation pendant 1 heure. Le catalyseur D ainsi dopé est ensuite rincé avec 10 ml de soude 3N, puis de soude 2N, puis de soude N et enfin 3 fois 10 ml d'eau. Il est stocké dans de la soude N.

Le catalyseur D a un rapport pondéral Cr/Ni de 1,5 %.

## Revendications

1. Procédé de préparation de catalyseurs d'hydrogénation de nitriles en amines, de type Nickel de Raney dopé par au moins un élément métallique d'addition, sélectionné parmi le chrome et le titane, caractérisé en ce qu'il consiste à mettre en suspension du Nickel de Raney dans une solution acide de l'élément métallique d'addition.

2. Procédé selon la revendications 1, caractérisé en ce que l'élément métallique d'addition mis en oeuvre se présente sous forme d'oxyde ou de sel d'acide minéral ou organique.

3. Procédé selon la revendication 2, caractérisé en ce que l'élément métallique d'addition se présente sous forme de chlorure et/ou d'oxyde, TiCl₃ étant particulièrement préféré.

4. Procédé selon l'une quelconque des revendications 1 à 3, caractérisé en ce que la mise en suspension est suivie d'un lavage, de préférence à l'eau, du nickel de Raney au moins en partie dopé par l'élément métallique d'addition, l'une au moins de ces deux opérations étant éventuellement renouvelée n fois, n étant avantageusement compris entre 1 et 5.

5. Procédé selon l'une quelconque des revendications 1 à 4, caractérisé en ce que le catalyseur Ni de Raney dopé est recueilli et stocké, de préférence, dans une base forte.

6. Procédé selon l'une quelconque des revendications 1 à 5, caractérisé en ce que l'on soumet le nickel de Raney, en suspension dans une solution d'au moins un élément métallique d'addition, à un traitement de lessivage alcalin, effectué à une température supérieure ou égale à la température ambiante, de préférence comprise entre 50°C et 200°C et plus préférentiellement encore comprise entre 80°C et 120°C, ce lessivage étant éventuellement effectué sous une pression en hydrogène comprise entre 0,1 et 20 MPa, de préférence, entre 1 et 5 MPa et plus préférentiellement encore entre 1,5 et 3 MPa.

7. Procédé selon l'une quelconque des revendications 1 à 6, caractérisé en ce que la quantité d'élément métallique d'addition mis en oeuvre et les conditions réactionnelles sont choisies de telle sorte que les catalyseurs obtenus aient un rapport dopant/Ni compris entre 0,5 % et 5 %, de préférence entre 1 % et 4 % et plus préférentiellement encore entre 1,2 % et 3 % en poids.

8. Procédé d'hydrogénation catalytique de nitriles en amines, caractérisé en ce qu'il consiste à recourir au catalyseur Ni de Raney dopé, obtenu par le procédé selon l'une quelconque des revendications 1 à 7.

9. Procédé selon la revendication 8, caractérisé en ce que l'on met en oeuvre un substrat nitrile de formule (I) :
NC―R―CN (I)
dans laquelle R représente un groupement alkylène ou alcénylène, linéaire ou ramifié, ayant de 1 à 12 atomes de carbone, ou un groupement arylène ou aralkylène ou aralcénylène substitué ou non,
et de préférence un nitrile de formule (I) dans laquelle R représente un radical alkylène, linéaire ou ramifié ayant de de 2 à 6 atomes de carbone.

10. Procédé selon la revendication 9, caractérisé en ce que le substrat nitrile est choisi parmi l'adiponitrile, le méthylglutaronitrile, l'éthylsuccinonitrile, le malononitrile, le succinonitrile et le glutaronitrile et leurs mélanges.

11. Procédé selon l'une quelconque des revendications 8 à 10, caractérisé en ce que le substrat, caractérisé en ce que l'on fixe la concentration en substrat nitrile dans le milieu réactionnel total à une valeur comprise entre 0,001 % et 30 % en poids par poids et de préférence entre 0,1 % et 20 %.

12. Procédé selon l'une quelconque des revendications 8 à 11, caractérisé en ce que l'on met en oeuvre une base constituée par au moins l'un des composés suivants : LiOH, NaOH, KOH, RbOH, CsOH.

13. Procédé selon l'une quelconque des revendications 8 à 12, caractérisé en ce que le milieu réactionnel liquide comprend de l'eau, de préférence dans une quantité inférieure ou égale à 20 % en poids du milieu réactionnel liquide total et, plus préférentiellement encore, comprise entre 0,1 % et 15 % en poids.

14. Procédé selon l'une quelconque des revendications 8 à 13, caractérisé en ce que le milieu réactionnel liquide contient de l'amine visée.

15. Procédé selon la revendication 14, caractérisé en ce que l'amine visée est introduite dans le milieu réactionnel liquide, à raison de 50 à 99 % et préférentiellement à raison de 60 à 99 % en poids par rapport au poids du milieu réactionnel liquide total.

16. Procédé selon l'une des revendications 8 à 15, caractérisé en ce que l'on met en oeuvre un milieu réactionnel liquide comprenant un alcool et/ou un amide.

17. Procédé selon la revendication 16, caractérisé en ce que l'alcool est sélectionné parmi les composés suivants : méthanol, éthanol, propanol, isopropanol, butanol, glycols tels que éthylèneglycol et/ou propylène glycol, polyols et leurs mélanges et en ce que l'amide est le diméthylformamide et/ou le diméthylacétamide.

18. Procédé selon l'une quelconque des revendications 8 à 15, caractérisé en ce que l'on utilise la base dans une quantité supérieure ou égale à 0,1 mol/kg de catalyseur, de préférence comprise entre 0,1 et 2,0 mol/kg de catalyseur et, plus préférentiellement encore, entre 0,5 et 1,5 mol/kg de catalyseur.

19. Procédé selon l'une quelconque des revendications 16 ou 17, caractérisé en ce que l'on utilise la base dans une quantité supérieure ou égale à 0,05 mol/kg de catalyseur, de préférence comprise entre 0,1 et 10,0 mol/kg et, plus préférentiellement encore, entre 1,0 et 8,0 mol/kg.

20. Procédé selon l'une quelconque des revendications 8 à 19, caractérisé en ce que l'on effectue l'hydrogénation à une température de milieu réactionnel inférieure ou égale à 150°C, de préférence inférieure ou égale à 120°C et, plus préférentiellement encore, inférieure ou égale à 100°C.

## Claims

1. Process for the preparation of catalysts for the hydrogenation of nitriles to amines of Raney nickel type doped with at least one additional metal element selected from chromium and titanium, characterized in that it consists in suspending Raney nickel in an acid solution of the additional metal element.

2. Process according to claim 1, characterized in that the additional metal element used is provided in the oxide or inorganic acid salt or organic acid salt form.

3. Process according to claim 2, characterized in that the additional metal element is provided in the chloride and/or oxide form, TiCl₃ being particularly preferred.

4. Process according to any one of claims 1 to 3, characterized in that the operation of placing in suspension is followed by a washing, preferably with water, of the Raney nickel at least partly doped with the additional metal element, at least one of these two operations optionally being repeated n times, n advantageously being between 1 and 5.

5. Process according to any one of claims 1 to 4, characterized in that the doped Raney Ni catalyst is collected and stored, preferably, in a strong base.

6. Process according to any one of claims 1 to 5, characterized in that the Raney nickel, suspended in a solution of at least one additional metal element, is subjected to an alkaline leaching treatment carried out at a temperature greater than or equal to room temperature, preferably between 50°C and 200°C and more preferentially still between 80°C and 120°C, this leaching optionally being carried out under a hydrogen pressure of between 0.1 and 20 MPa, preferably between 1 and 5 MPa and more preferentially still between 1.5 and 3 MPa.

7. Process according to any one of claims 1 to 6, characterized in that the amount of additional metal element used and the reaction conditions are chosen so that the catalysts obtained have a doping agent/Ni ratio of between 0.5% and 5%, preferably between 1% and 4% and more preferentially still between 1.2% and 3% by weight.

8. Process for the catalytic hydrogenation of nitriles to amines, characterized in that it consists in resorting to the doped Raney Ni catalyst obtained by the process according to any one of claims 1 to 7.

9. Process according to claim 8, characterized in that use is made of a nitrile substrate of formula (I):
NC-R-CN (I)
in which R represents a linear or branched alkylene or alkenylene group having from 1 to 12 carbon atoms or a substituted or unsubstituted arylene or aralkylene or aralkenylene group,
and preferably a nitrile of formula (I) in which R represents a linear or branched alkylene radical having from 2 to 6 carbon atoms.

10. Process according to claim 9, characterized in that the nitrile substrate is chosen from adiponitrile, methylglutaronitrile, ethylsuccinonitrile, malononitrile, succinonitrile and glutaronitrile and their mixtures.

11. Process according to any one of claims 8 to 10, characterized in that the concentration of nitrile substrate in the total reaction medium is set at a value of between 0.001% and 30% weight for weight and preferably between 0.1% and 20%.

12. Process according to any one of claims 8 to 11, characterized in that use is made of a base consisting of at least one of the following compounds: LiOH, NaOH, KOH, RbOH, CsOH.

13. Process according to any one of claims 8 to 12, characterized in that the liquid reaction medium comprises water, preferably in an amount less than or equal to 20% by weight of the total liquid reaction medium and, more preferentially still, between 0.1% and 15% by weight.

14. Process according to any one of claims 8 to 13, characterized in that the liquid reaction medium contains targeted amine.

15. Process according to claim 14, characterized in that the targeted amine is introduced into the liquid reaction medium in a proportion of 50 to 99% and preferentially in a proportion of 60 to 99% by weight with respect to the weight of the total liquid reaction medium.

16. Process according to any one of claims 8 to 15, characterized in that use is made of a liquid reaction medium comprising an alcohol and/or an amide.

17. Process according to claim 16, characterized in that the alcohol is selected from the following compounds: methanol, ethanol, propanol, isopropanol, butanol, glycols, such as ethylene glycol and/or propylene glycol, polyols and their mixtures and in that the amide is dimethylformamide and/or dimethylacetamide.

18. Process according to any one of claims 8 to 15, characterized in that the base is used in an amount greater than or equal to 0.1 mol/kg of catalyst, preferably between 0.1 and 2.0 mol/kg of catalyst and, more preferentially still, between 0.5 and 1.5 mol/kg of catalyst.

19. Process according to either of claims 16 and 17, characterized in that the base is used in an amount greater than or equal to 0.05 mol/kg of catalyst, preferably between 0.1 and 10.0 mol/kg and, more preferentially still, between 1.0 and 8.0 mol/kg.

20. Process according to any one of claims 8 to 19, characterized in that the hydrogenation is carried out at a temperature of the reaction medium which is less than or equal to 150°C, preferably less than or equal to 120°C and, more preferentially still, less than or equal to 100°C.

## Patentansprüche

1. Verfahren zur Herstellung von Katalysatoren vom Typ Raney-Nickel führ die Hydrierung von Nitrilen zu Aminen, dotiert durch mindestens ein metallisches Zusatzelement, ausgewählt unter Chrom und Titan, dadurch gekennzeichnet, daß es darin besteht, das Raney-Nickel in einer sauren Lösung des metallischen Zusatzelementes in Suspension zu bringen.

2. Verfahren nach Anspruch 1, dadurch gekennzeichnet, daß das eingesetzte metallische Zusatzelement in Form von Oxid oder Salz einer Mineralsäure oder organischen Säure vorliegt.

3. Verfahren nach Anspruch 2, dadurch gekennzeichnet, daß das metallische Zusatzelement in Form von Chlorid und/oder Oxid vorliegt, wobei TiCl₃ besonders bevorzugt ist.

4. Verfahren nach irgendeinem der Ansprüche 1 bis 3, dadurch gekennzeichnet, daß dem Suspendieren ein Waschen, vorzugsweise mit Wasser, des mindestens teilweise mit dem metallischen Zusatzelement dotierten Raney-Nickels folgt, wobei mindestens eine von diesen zwei Operationen gegebenenfalls n mal wiederholt wird und n vorteilhafterweise zwischen 1 und 5 beträgt.

5. Verfahren nach irgendeinem der Ansprüche 1 bis 4, dadurch gekennzeichnet, daß der dotierte Katalysator aus Raney-Nickel vorzugsweise in einer starken Base aufgefangen und gelagert wird.

6. Verfahren nach irgendeinem der Ansprüche 1 bis 5, dadurch gekennzeichnet, daß man das sich in Suspension in einer Lösung von mindestens einem metallischen Zusatzelement befindende Raney-Nickel einer alkalischen Auslaugungs-Behandlung unterzieht, durchgeführt bei einer Temperatur von höher oder gleich der Umgebungstemperatur, vorzugsweise bei einer Temperatur zwischen 50 °C und 200 °C und noch bevorzugter zwischen 80 °C und 120 °C, wobei dieses Auslaugen gegebenenfalls unter einem Wasserstoffdruck zwischen 0,1 MPa und 20 MPa, vorzugsweise zwischen 1 MPa und 5 MPa und noch bevorzugter zwischen 1,5 MPa und 3 MPa vorgenommen wird.

7. Verfahren nach irgendeinem der Ansprüche 1 bis 6, dadurch gekennzeichnet, daß die Menge des eingesetzten metallischen Zusatzelementes und die Reaktionsbedingungen in der Weise gewählt werden, daß die erhaltenen Katalysatoren ein Verhältnis Dotierungs-Element/Ni zwischen 0,5 Gew.-% und 5 Gew.-%, vorzugsweise zwischen 1 Gew.-% und 4 Gew.-% und noch bevorzugter zwischen 1,2 Gew.-% und 3 Gew.-% aufweisen.

8. Verfahren zur katalytischen Hydrierung von Nitrilen zu Aminen, dadurch gekennzeichnet, daß es darin besteht, einen Katalysator aus dotiertem Raney-Nickel einzusetzen, erhalten durch das Verfahren nach einem der Ansprüche 1 bis 7.

9. Verfahren nach Anspruch 8, dadurch gekennzeichnet, daß man ein Nitrilsubstrat der Formel (I)
NC-R-CN (I)
einsetzt, in der R eine gerade oder verzweigte Gruppe Alkylen oder Alkenylen mit 1 bis 12 Kohlenstoffatomen oder eine substituierte oder nicht substituierte Gruppe Arylen oder Aralkylen oder Aralkenylen darstellt, und vorzugsweise ein Nitril der Formel (I) verwendet, in der R einen geraden oder verzweigten Rest Alkylen mit 2 bis 6 Kohlenstoffatomen bedeutet.

10. Verfahren nach Anspruch 9, dadurch gekennzeichnet, daß das Nitrilsubstrat unter Adiponitril, Methylglutaronitril, Ethylsuccinonitril, Malononitril, Succinonitril und Glutaronitril sowie deren Mischungen ausgewählt wird.

11. Verfahren nach irgendeinem der Ansprüche 8 bis 10, dadurch gekennzeichnet, daß das Substrat dadurch gekennzeichnet ist, daß man die Konzentration an Nitrilsubstrat in dem gesamten Reaktionsmilieu auf einen Wert zwischen 0,001 Gew.-% und 30 Gew.-% pro Gewicht und vorzugsweise zwischen 0,1 % und 20 % festlegt.

12. Verfahren nach irgendeinem der Ansprüche 8 bis 11, dadurch gekennzeichnet, daß man eine Base einsetzt, bestehend aus mindestens einer der folgenden Verbindungen: LiOH, NaOH, KOH, RbOH, CsOH.

13. Verfahren nach irgendeinem der Ansprüche 8 bis 12, dadurch gekennzeichnet, daß das flüssige Reaktionsmilieu Wasser umfaßt, vorzugsweise in einer Menge von unterhalb oder gleich 20 Gew.-% des gesamten flüssigen Reaktionsmilieus und noch bevorzugter zwischen 0,1 Gew.-% und 15 Gew.-%.

14. Verfahren nach irgendeinem der Ansprüche 8 bis 13, dadurch gekennzeichnet, daß das flüssige Reaktionsmilieu das angestrebte Amin enthält.

15. Verfahren nach Anspruch 14, dadurch gekennzeichnet, daß das angestrebte Amin in das flüssige Reaktionsmilieu in einem Verhältnis von 50 Gew.-% bis 99 Gew.-% und vorzugsweise in einem Verhältnis von 60 Gew.-% bis 99 Gew.-%, bezogen auf das Gewicht des gesamten flüssigen Reaktionsmilieu, eingetragen wird.

16. Verfahren nach einem der Ansprüche 8 bis 15, dadurch gekennzeichnet, daß man ein flüssiges Reaktionsmilieu einsetzt, das einen Alkohol und/oder ein Amid umfaßt.

17. Verfahren nach Anspruch 16, dadurch gekennzeichnet, daß der Alkohol unter den folgenden Verbindungen: Methanol, Ethanol, Propanol, Isopropanol, Butanol, Glycolen wie Ethylenglycol und/oder Propylenglycol, Polyolen und ihren Mischungen ausgewählt wird, und daß das Amid Dimethylformamid und/oder Dimethylacetamid ist.

18. Verfahren nach irgendeinem der Ansprüche 8 bis 15, dadurch gekennzeichnet, daß man die Base in einer Menge von höher oder gleich 0,1 Mol/kg Katalysator, vorzugsweise zwischen 0,1 Mol/kg und 2,0 Mol/kg Katalysator und noch bevorzugter zwischen 0,5 Mol/kg und 1,5 Mol/kg Katalysator verwendet.

19. Verfahren nach irgendeinem der Ansprüche 16 oder 17, dadurch gekennzeichnet, daß man die Base in einer Menge von höher oder gleich 0,05 Mol/kg Katalysator, vorzugsweise zwischen 0,1 Mol/kg und 10,0 Mol/kg und noch bevorzugter zwischen 1,0 Mol/kg und 8,0 Mol/kg Katalysator verwendet.

20. Verfahren nach irgendeinem der Ansprüche 8 bis 19, dadurch gekennzeichnet, daß man die Hydrierung bei einer Temperatur des Reaktionsmilieus von unterhalb oder gleich 150 °C, vorzugsweise von unterhalb oder gleich 120 °C und noch bevorzugter von unterhalb oder gleich 100 °C durchführt.
